# EUROPEAN PATENT SPECIFICATION

(11) **EP 2 107 916 B1**
(45) Date of publication and mention of the grant of the patent: **29.06.2011**
(21) Application number: 08708582.5
(22) Date of filing: 01.02.2008
(51) Int. Cl.: A61M 5/158, A61M 5/162, A61M 39/02

(54) **A GATEWAY DEVICE**
GATEWAY-VORRICHTUNG
DISPOSITIF PASSERELLE

(30) Priority: 02.02.2007 DK 200700185; 02.02.2007 DK 200700191; 02.02.2007 DK 200700179; 02.02.2007 US 899075 P; 02.02.2007 US 899062 P; 02.02.2007 US 899143 P
(43) Date of publication of application: 14.10.2009
(73) Proprietor: Unomedical A/S, 3460 Birkerød (DK)
(72) Inventor: GRUNDTVIG THEANDER, Julie, DK-3650 Ølstykke (DK); BOYUM HAGEDORN, Brian, DK-4180 Sorø (DK)
(74) Representative: Sundien, Thomas
(86) International application number: PCT/EP2008/051276
(87) International publication number: WO 2008/092958

(56) References cited:
- WO-A-02/081013
- WO-A-2004/037325
- WO-A-2004/064593
- US-A1- 2002 072 720
- US-A1- 2005 107 743

## Description

The present invention relates to a gateway device for the delivery of a drug, the device comprising a cannula for delivery of said drug, a housing having a base adapted for being placed against the skin of a patient, and a receiving area for receiving a coupling portion of a drug supply device for supplying the drug to the gateway device, the receiving area including a hollow tubular coupling having a free end and being for engaging the coupling portion, the hollow tubular coupling being arranged for delivery of said drug to said patient via the cannula. the cannula being secured to the housing to allow the cannula to project from said housing.

Conventionally, gateway devices for subcutaneous delivery of a drug require .. an insertion needle which is passed through the cannula and which is used for placing the gateway device on the patient. This involves the problem that a complicated gateway structure with a separate membrane or septum is required for sealing off the entry port of the insertion needle.

The above problem is solved through the invention by providing a hollow tubular member with a first end portion and a second end portion that defines the cannula and the tubular coupling such the hollow tubular member not only serves as a cannula for delivery of drug but also serves as an insertion needle allowing the user to use the first end portion for inserting the gateway device into the skin whereby no separate insertion needle is required for placing the gateway device. Hence, the first end portion serves the dual function of a cannula and of an insertion needle for the first time placement of the device, the free end of the tubular coupling preferably being adapted for piercing or penetrating a septum of the coupling portion.

Embodiments of the invention will now be discussed with reference to the drawings.
Fig. 1 is a perspective view seen from above of one embodiment of the device according to the invention,
Fig. 2 is a view similar to fig. 1, with a cap mounted on the device,
Fig. 3 is a cross-sectional view of the device as shown in fig. 2, and
Fig. 4a and 4b are schematic side views of second and third embodiments of the device.

In use of a gateway device of the invention, after placement of the gateway device on a patient, the gateway device is coupled to a drug supply device that has a coupling portion suitable for coupling the drug supply device to the gateway to supply a drug to the patient via the gateway device.

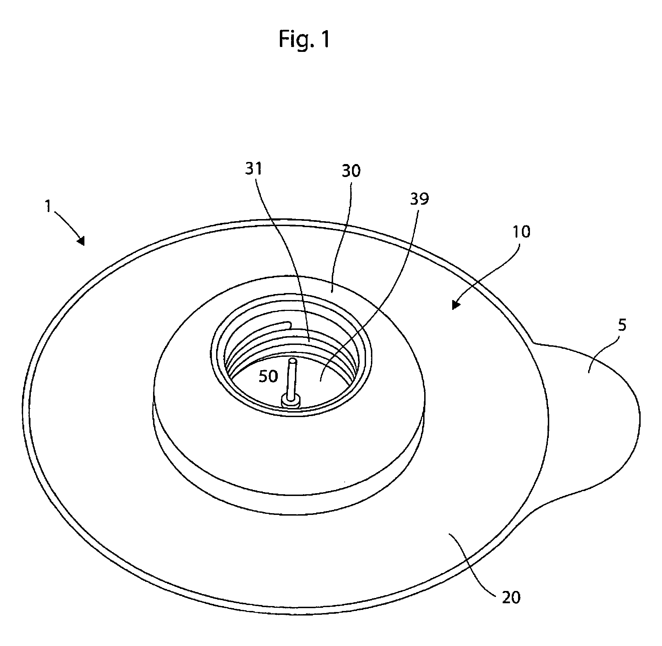

Fig. 1 shows a first embodiment of a gateway device according to the invention. The gateway device 1 comprises a housing 10 with a base 20 with a lower surface which in use of the gateway device 1 is placed and maintained in position against the skin of a patient to which the drug, such as insulin, is to be administered. The housing 10 may be molded in its entirety from a plastics material, or the device preferably may have an upper housing part molded of a plastics material and to which a flexible sheet lower housing part defining the base 20 may be attached, such as by way of an adhesive. The base 20 lower surface preferably has an adhesive layer covered by a releasable protective sheet 5.

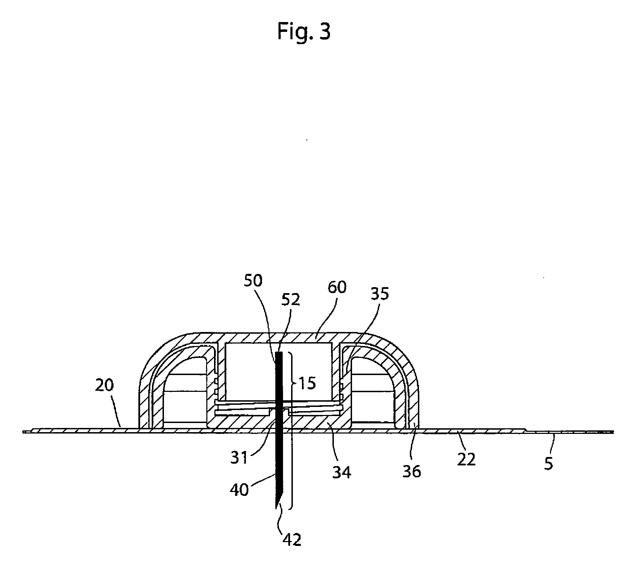

The gateway device 1 has a cannula 40 for delivery of the drug, the cannula 40 with free end 42 being secured to the housing to allow the cannula 40 to project from the housing 10, preferably such that the cannula 40 extends from the lower surface 2 (see fig. 3) of the base, perpendicularly or at an angle thereto; alternatively the cannula 40 may extend from other parts of the housing.

The housing 10 also includes a receiving area 30 with a central recess 39 for receiving the coupling portion of the aforementioned drug supply device, and the receiving area 30 centrally therein has a hollow tubular coupling 50 that has a free end 52 and is for engaging the coupling portion of the drug supply device. Often the coupling portion of the drug supply device will have a septum, and when the free end 52 of the hollow tubular coupling penetrates the septum by the user inserting the drug supply device coupling portion into the recess 39 the drug will flow to the patient via the cannula 40 which previously has been placed subcutaneously.

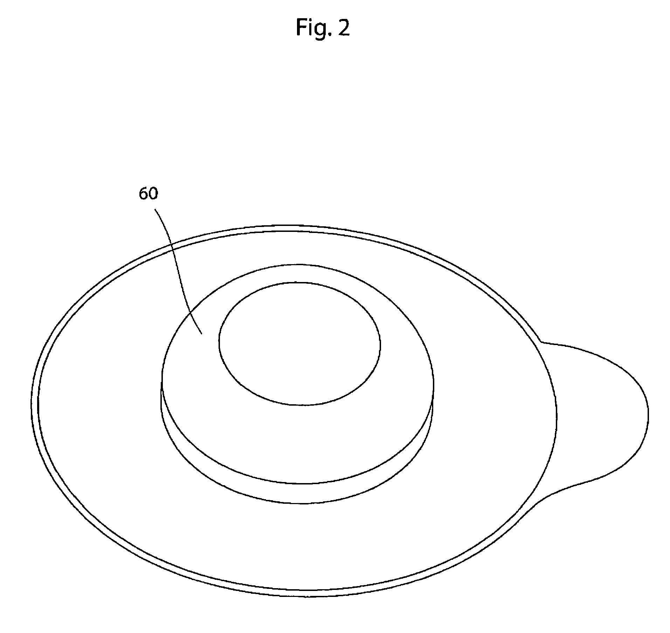

The free end 52 of the tubular coupling 50 is preferably sharpened allowing for an easy penetration of the septum, and the tubular coupling 50 may not project beyond the recess 39, As shown in fig. 2, the device preferably includes a releasable and repositionable cap or cover 60, which may define a microbial barrier, for covering the sharpened free end 52 of the tubular coupling 50 in order to protect the user against inadvertent sticking himself on the tubular coupling 50. The cover 60 is preferably molded from a plastics material and may be connected to the housing 10 in any appropriate manner, such as by way of a screw-thread 31 as illustrated schematically in fig. 1, or by cooperating engagement means providing for a snap-engagement between the cover 60 and the housing 10. Alternatively a flexible sheet cover 60 may be adhered to the housing 10.

Fig. 3 shows the device with cover 60, a circumferential wall 35 defining the recess 39 and being integral with a recess bottom wall 34 and an outer circumferential wall 36.

The hollow tubular coupling 50 is defined by a first end portion of a hollow tubular member while the cannula 40 is defined by the opposite, second end portion of the hollow tubular member. The hollow tubular member is referenced by numeral 15 in fig. 3, and the tubular member 15 has a middle portion fixed in the housing 10 in an area of the recess bottom wall 34 designated reference numeral 31, to keep the tubular member 15 in position with respect to the housing 10. In use, the drug flows through and along the inside of the hollow tubular member 15 between the ends 42, 52 thereof.

The hollow tubular member 15 serves several purposes. The first end portion of the hollow tubular member 15 not only serves as a cannula for delivery of drug but also serves as an insertion needle allowing the user to use the first end portion for inserting the gateway device 1 into the skin, such that no separate insertion needle is required for placing the device 1. Hence, the first end portion serves the dual function of a cannula and of an insertion needle for the first time placement of the device. The cannula defined by the first end portion may be more flexible than the tubular coupling defined by the second end portion of the hollow tubular member 15, to increase the comfort experienced by a patient using the gateway device 1, and the tip portion or free end 42 of the cannula 40 may be sharpened. To increase flexibility the first end portion of the hollow tubular member 15 may have reduced cross-sectional dimensions, such as wall-thickness, or the first end portion may be formed from a material different from the second end portion. The hollow tubular member 15 may be formed from the same material, such as steel, and may have uniform cross-sectional dimensions along the length, until the sharpened tip-portions 42, 52 thereof.

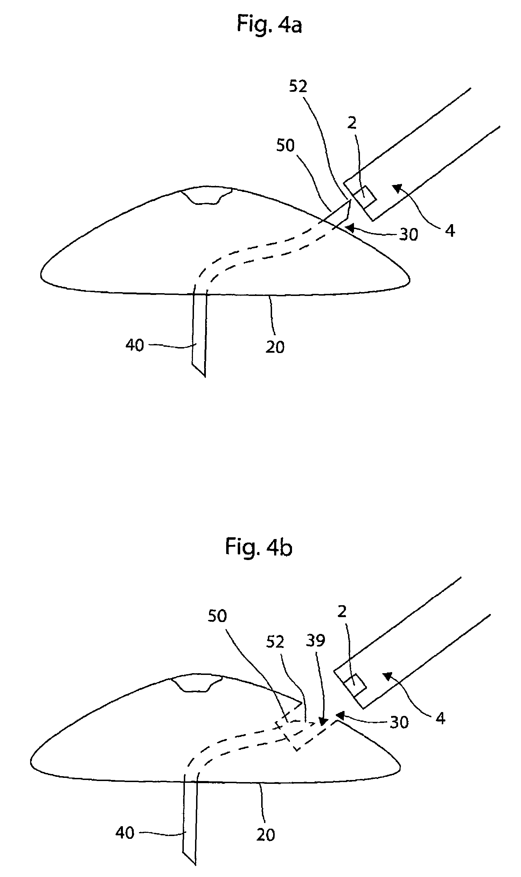

As will be seen from fig. 3 the hollow tubular member is preferably a one-piece member defining a non-leaking flow path for drug to be administered, and the tubular member 15 may be straight as in fig. 3, or may assume a bent or curved configuration as illustrated schematically in fig. 4a and 4b where the tubular coupling 50 extends at an angle with respect to the cannula 40 at the free end 52 to allow the drug supply device with coupling portion septum 2 to be supported by the coupling portion receiving area 30 at an angle to the base 20. In fig. 4a and 4b the hollow tubular member 15 is shaped as an S to provide a curved flow path between the drug supply device 4 and the patient. In fig. 4b, a recess 39 is provided to provide lateral support and guiding for the supply device such that the hollow tubular coupling 50 is at less risk of being damaged.

While in figs. 1-3 a well-defined receiving area formed as a recess is shown, the invention is by no way limited to the gateway device housing having any specific receiving area defining structure. Thus, in principle the receiving area may simply be a surface area of the housing with the tubular coupling extending upwards therefrom, as shown in fig. 4a, and the receiving area may or may not be adapted to provide for support for the supply device.

## Claims

1. A gateway device (1) for the delivery of a drug, said device comprising:
a cannula (40) for delivery of said drug,
a housing (10) having
- a base (20) adapted for being placed against the skin of a patient, and
- a receiving area (30) for receiving a coupling portion (2) of a drug supply device (4) for supplying said drug to said gateway device,
-- said receiving area (30) including a hollow tubular coupling (50) having a free end (52) and being for engaging said coupling portion,
-- said hollow tubular coupling (50) being arranged for delivery of said drug to said patient via said cannula (40),
said cannula (40) being secured to said housing (10) to allow said cannula (40) to project from said housing (10),
**characterised in** a hollow tubular member with a first end portion and a second end portion defines said cannula and said tubular coupling.

2. The gateway according to claim 1, said free end (52) of said tubular coupling being adapted for piercing or penetrating a septum (2) of said coupling portion.

3. The gateway according to any of the preceding claims, said cannula being more flexible than said tubular coupling.

4. The gateway according to any of the preceding claims, said cannula being made from a plastics material and said tubular coupling being metallic.

5. The gateway according to any of the preceding claims 1-3, said cannula and said tubular coupling being of the same material.

6. The gateway according to any of the preceding claims, said cannula (40) and said hollow tubular coupling (50) together defining a non-leaking flow path for said drug.

7. The gateway according to any of the preceding claims, said hollow tubular member being a needle.

8. The gateway according to any of the preceding claims, said hollow tubular member assuming a straight configuration.

9. The gateway according to any of the preceding claims 1-7, said hollow tubular member assuming a bend configuration.

10. The gateway according to any of the preceding claims, said tubular coupling (50) being opposite to said receiving area (30) and extending perpendicularly to said base.

11. The gateway according to any of the preceding claims 1-9, said tubular coupling being opposite to said receiving area (30) and extending at an angle to said base (20).

12. The gateway according to any of the preceding claims, said housing including a releasable cover (60) for covering said tubular coupling (50).

13. The gateway according to the preceding claim, said cover surrounding said receiving area (30).

14. The gateway according to claim 12 or 13, said cover (60) being secured to said receiving area by a screw-thread 31, or by snap engagement.

15. The gateway according to any of claims 12-14, said cover being hinged to said receiving area.

16. The gateway according to any of claims 12-15, said receiving area including a wall structure (35) surrounding said tubular coupling (50).

17. The gateway device according to claim 16, said cover (60) being secured to said wall structure (35).

18. The gateway device according to any of claims 16 or 17, an imaginary extension of said wall structure (35) below said base (20) surrounding said cannula (40).

19. The gateway according to any of the preceding claims, said receiving area (30) including a recess (39) with said tubular coupling (50).

20. The gateway according to any of the preceding claims, said tubular coupling not projecting from said receiving area (30).

21. The gateway according to any of the preceding claims, said receiving area (30) being integrally formed with said base (20), such as by molding.

22. The gateway according to any of the preceding claims, said base (20) including an adhesive layer for securing said base to said patient, said device including releasable protection (5) on said adhesive layer.

23. The gateway according to any of the preceding claims, said cannula (40) projecting from said base (20).

24. The gateway according to any one of the preceding claims, said hollow-tubular member being a one-piece member.

25. A system comprising the gateway device of any of the preceding claims, and a drug supply device with a coupling portion for supplying said drug to said gateway device, said coupling portion including a septum adapted to be pierced or penetrated by said hollow tubular coupling.

## Patentansprüche

1. Gateway-Vorrichtung (1) für die Anlieferung eines Medikaments, welche Vorrichtung umfasst:
eine Kanüle (40) für Anlieferung des Medikaments,
ein Gehäuse (10), aufweisend
- eine Basis (20), die für die Platzierung gegen die Haut eines Patienten angepasst ist,
- einen Aufnahmebereich (30) zur Aufnahme eines Koppelungsteils (2) einer Medikamentenzuführvorrichtung (4) zum Zuführen des Medikaments zur Gateway-Vorrichtung,
-- welcher Aufnahmebereich (30) eine hohle rohrförmige Koppelung (50) enthält, die ein freies Ende (52) aufweist und für die Verbindung des Koppelungsteils vorgesehen ist,
-- welche hohle rohrförmige Koppelung (50) über die Kanüle (40) für die Anlieferung des Medikaments an den Patienten vorgesehen ist,
welche Kanüle (40) am Gehäuse (10) gesichert ist, um das Herausragen der Kanüle (40) aus dem Gehäuse (10) zu erlauben,
**dadurch gekennzeichnet, dass** ein hohles rohrförmiges Element mit einem ersten Endteil und einem zweiten Endteil die Kanüle und die rohrförmige Koppelung definiert.

2. Gateway nach Anspruch 1, wobei das freie Ende (52) der rohrförmigen Koppelung für das Durchdringen oder die Penetration eines Septums (2) des Koppelungsteils angepasst ist.

3. Gateway nach irgendeinem der vorhergehenden Ansprüche, wobei die Kanüle flexibler ist als die rohrförmige Koppelung.

4. Gateway nach irgendeinem der vorhergehenden Ansprüche, wobei die Kanüle aus einem Plastikmaterial hergestellt ist, und die rohrförmige Koppelung metallisch ist.

5. Gateway nach irgendeinem der vorhergehenden Ansprüche 1-3, wobei die Kanüle und die rohrförmige Koppelung aus gleichem Material sind.

6. Gateway nach irgendeinem der vorhergehenden Ansprüche, wobei die Kanüle (40) und die hohle rohrförmige Koppelung (50) zusammen einen nicht-leckenden Strömungsweg für das Medikament definieren.

7. Gateway nach irgendeinem der vorhergehenden Ansprüche, wobei das hohle rohrförmige Element eine Nadel ist.

8. Gateway nach irgendeinem der vorhergehenden Ansprüche, wobei das hohle rohrförmige Element eine gerade Konfiguration annimmt.

9. Gateway nach irgendeinem der vorhergehenden Ansprüche 1-7, wobei das hohle rohrförmige Element eine Biegungskonfiguration annimmt.

10. Gateway nach irgendeinem der vorhergehenden Ansprüche, wobei die rohrförmige Koppelung (50) dem Aufnahmebereich (30) entgegengesetzt ist und sich senkrecht zur Basis erstreckt.

11. Gateway nach irgendeinem der vorhergehenden Ansprüche 1-9, wobei die rohrförmige Koppelung dem Aufnahmebereich (30) entgegengesetzt ist und sich um einen Winkel zur Basis (20) erstreckt.

12. Gateway nach irgendeinem der vorhergehenden Ansprüche, wobei das Gehäuse eine freisetzbare Abdeckung (60) zur Deckung der rohrförmigen Koppelung (50) enthält.

13. Gateway nach dem vorhergehenden Anspruch, wobei die Abdeckung den Aufnahmebereich (30) umgibt.

14. Gateway nach Anspruch 12 oder 13, wobei die Abdeckung (60) durch ein Schraubengewinde 31 oder durch einen Schnappeingriff am Aufnahmebereich gesichert ist.

15. Gateway nach irgendeinem der Ansprüche 12-14, wobei die Abdeckung am Aufnahmebereich angelenkt ist.

16. Gateway nach irgendeinem der Ansprüche 12-15, wobei der Aufnahmebereich eine die rohrförmige Koppelung (50) umgebende Wandstruktur (35) enthält.

17. Gateway-Vorrichtung nach Anspruch 16, wobei die Abdeckung (60) an der Wandstruktur (35) gesichert ist.

18. Gateway-Vorrichtung nach irgendeinem der Ansprüche 16 oder 17, wobei eine imaginäre Verlängerung der Wandstruktur (35) unterhalb der Basis (20) die Kanüle (40) umgibt.

19. Gateway nach irgendeinem der vorhergehenden Ansprüche, wobei der Aufnahmebereich (30) eine Ausnehmung (39) mit der rohrförmigen Koppelung (50) enthält.

20. Gateway nach irgendeinem der vorhergehenden Ansprüche, wobei die rohrförmige Koppelung nicht aus dem Aufnahmebereich (30) herausragt.

21. Gateway nach irgendeinem der vorhergehenden Ansprüche, wobei der Aufnahmebereich (30) wie etwa durch Formung mit der Basis (20) ganz ausgebildet ist.

22. Gateway nach irgendeinem der vorhergehenden Ansprüche, wobei die Basis (20) eine anhaftende Schicht zur Sicherung der Basis am Patienten enthält, wobei die Vorrichtung einen freisetzbaren Schutz (5) auf der anhaftenden Schicht enthält.

23. Gateway nach irgendeinem der vorhergehenden Ansprüche, wobei die Kanüle (40) aus der Basis (20) herausragt.

24. Gateway nach irgendeinem der vorhergehenden Ansprüche, wobei das hohl-rohrförmige Element ein einstückiges Element ist.

25. System, umfassend die Gateway-Vorrichtung nach irgendeinem der vorhergehenden Ansprüche, und eine Medikamentenzuführvorrichtung mit einem Koppelungsteil zum Zuführen des Medikaments zur Gateway-Vorrichtung, wobei der Koppelungsteil ein Septum enthält, das dafür angepasst ist, von der hohlen rohrförmigen Koppelung durchdrungen oder penetriert zu werden.

## Revendications

1. Dispositif passerelle (1) pour la délivrance d'un médicament, ledit dispositif comprenant:
une canule (40) pour la délivrance dudit médicament,
un boîtier (10) présentant
- une base (20) adaptée à être placée contre la peau d'un patient, et
- une zone de réception (30) pour recevoir une partie d'accouplement (2) d'un dispositif d'alimentation de médicament (4) pour la délivrance dudit médicament audit dispositif de passerelle,
-- ladite zone de réception (30) comprenant un accouplement tubulaire creux (50) présentant une extrémité libre (52) et étant adapté à engager ladite partie d'accouplement,
-- ledit accouplement tubulaire creux (50) étant arrangé pour la délivrance dudit médicament audit patient par l'intermédiaire de ladite canule (40),
ladite canule (40) étant fixée audit boitier (10) pour permettre à ladite canule (40) de faire saillie dudit boîtier (10),
**caractérisé en ce qu'**un élément tubulaire creux avec une première partie d'extrémité et une deuxième partie d'extrémité définit ladite canule et ledit accouplement tubulaire.

2. Passerelle selon la revendication 1, ladite extrémité libre (52) dudit accouplement tubulaire étant adaptée à percer ou pénétrer un septum (2) de ladite partie d'accouplement.

3. Passerelle selon l'une quelconque des revendications précédentes, ladite canule étant plus flexible que ledit accouplement tubulaire.

4. Passerelle selon l'une quelconque des revendications précédentes, ladite canule étant faite de matière plastique et ledit accouplement tubulaire étant métallique.

5. Passerelle selon l'une quelconque des revendications précédentes 1 à 3, ladite canule et ledit accouplement tubulaire étant du même matériau.

6. Passerelle selon l'une quelconque des revendications précédentes, ladite canule (40) et ledit accouplement tubulaire creux (50) définissant conjointement un trajet d'écoulement sans fuite pour ledit médicament.

7. Passerelle selon l'une quelconque des revendications précédentes, ledit élément tubulaire creux étant une aiguille.

8. Passerelle selon l'une quelconque des revendications précédentes, ledit élément tubulaire creux présentant une configuration droite.

9. Passerelle selon l'une quelconque des revendications précédentes 1 à 7, ledit élément tubulaire creux présentant une configuration courbée.

10. Passerelle selon l'une quelconque des revendications précédentes, ledit accouplement tubulaire (50) étant opposé à ladite zone de réception (30) et s'étendant perpendiculairement à ladite base.

11. Passerelle selon l'une quelconque des revendications précédentes 1 à 9, ledit accouplement tubulaire étant opposé à ladite zone de réception (30) et s'étendant à un angle à ladite base (20).

12. Passerelle selon l'une quelconque des revendications précédentes, ledit boîtier comprenant un couvercle libérable (60) pour couvrir ledit accouplement tubulaire (50).

13. Passerelle selon la revendication précédente, ledit couvercle entourant ladite zone de réception (30).

14. Passerelle selon la revendication 12 ou 13, ledit couvercle (60) étant fixé à ladite zone de réception par un filet de vis (31) ou par prise à enclenchement.

15. Passerelle selon l'une quelconque des revendications 12 à 14, ledit couvercle étant articulé à ladite zone de réception.

16. Passerelle selon l'une quelconque des revendications 12 à 15, ladite zone de réception comprenant une structure de paroi (35) entourant ledit accouplement tubulaire (50).

17. Dispositif passerelle selon la revendication 16, ledit couvercle (60) étant fixé à ladite structure de paroi (35).

18. Dispositif de passerelle selon l'une quelconque des revendications 16 ou 17, un prolongement imaginaire de ladite structure de paroi (35) au-dessous de ladite base (20) entourant ladite canule (40).

19. Passerelle selon l'une quelconque des revendications précédentes, ladite zone de réception (30) comprenant une cavité (39) avec ledit accouplement tubulaire (50).

20. Passerelle selon l'une quelconque des revendications précédentes, ledit accouplement tubulaire ne faisant pas saillie depuis ladite zone de réception (30).

21. Passerelle selon l'une quelconque des revendications précédentes, ladite zone de réception (30) étant faite d'une pièce avec ladite base (20), p. ex. par moulage.

22. Passerelle selon l'une quelconque des revendications précédentes, ladite base (20) comprenant une couche adhésive pour fixer ladite base audit patient, ledit dispositif comprenant une protection libérable (5) sur ladite couche adhésive.

23. Passerelle selon l'une quelconque des revendications précédentes, ladite canule (40) faisant saillie depuis ladite base (20).

24. Passerelle selon l'une quelconque des revendications précédentes, ledit élément tubulaire et creux étant un élément d'une seule pièce.

25. Système comprenant le dispositif passerelle selon l'une quelconque des revendications précédentes, et un dispositif d'alimentation de médicament avec une partie d'accouplement pour fournir ledit médicament audit dispositif passerelle, ladite partie d'accouplement comprenant un septum adapté à être percé ou pénétré par ledit accouplement tubulaire creux.
